# EUROPEAN PATENT APPLICATION

(11) **EP 4 006 150 A1**
(43) Date of publication of application: **01.06.2022**
(21) Application number: 21175895.8
(22) Date of filing: 29.08.2016
(51) Int. Cl.: C12N 9/12, C12N 15/62

(54) **POLYMERASE ENZYME**

(30) Priority: 09.09.2015 EP 15184470
(62) Divisional of application: 16757670.1
(71) Applicant: QIAGEN GmbH, 40724 Hilden (DE)
(72) Inventor: Peist, Ralf, 40723 Hilden (DE); NARZ, Frank, 58456 Witten (DE); GRASSE, Nicole, 42781 Haan (DE); SPRIESTERSBACH, Anne, 50735 Köln (DE); RÜTJES, Thea, 42697 Solingen (DE)
(74) Representative: Cooley (UK) LLP

(57) **Abstract**

The present invention relates to a family B polymerase enzyme comprising the following mutations, a mutation in the motif A region, wherein a threonine is the second amino acid of the motif A region, wherein the motif A region is homologous to amino acids 408 to 410 in 9°N, a mutation leading to a reduction or elimination of the 3'-5' exonuclease activity if present in said family B polymerase, wherein said polymerase additionally comprises one or both of the following mutations in the motif A region, L408Y or L408F and/or P410S or P410G. It may be used in DNA sequencing and may be present in a kit.

## Description

### FIELD OF THE INVENTION

The present invention is in the field of molecular biology, in particular in the field of enzymes and more particular in the field of polymerases. It is also in the field of nucleic acid sequencing.

### BACKGROUND

The invention relates to polymerase enzymes, in particular modified DNA polymerases, which show improved incorporation of modified nucleotides compared to a control polymerase. Also included in the present invention are methods of using the modified polymerases for DNA sequencing, in particular next generation sequencing.

Three main super families of DNA polymerase exist, based upon their amino acid similarity to *E. coli* DNA polymerases I, II and III. They are called family A, B and C polymerases respectively. Whilst crystallographic analysis of Family A and B polymerases reveals a common structural core for the nucleotide binding site, sequence motifs that are well conserved within families are only weakly conserved between families, and there are significant differences in the way these polymerases discriminate between nucleotide analogues. Early experiments with DNA polymerases revealed difficulties incorporating modified nucleotides such as dideoxynucleotides (ddNTPs). There are, therefore, several examples in which DNA polymerases have been modified to increase the rates of incorporation of nucleotide analogues. The majority of these have focused on variants of Family A polymerases with the aim of increasing the incorporation of dideoxynucleotide chain terminators. For example, Taborand Richardson (Proc. Natl. Acad. Sci. (USA) 1995; 92:6339) describe the replacement of phenylalanine 667 with tyrosine in *T. aquaticus* DNA polymerase and the effects this has on discrimination of dideoxynucleotides by the DNA polymerase.

In order to increase the efficiency of incorporation of modified nucleotides, DNA polymerases have been utilised or engineered such that they lack 3' -5' exonuclease activity (designated exo⁻). The exo⁻ variant of 9°N polymerase is described by Perler et al., 1998 in US 5756334 and by Southworth et al., Proc. Natl. Acad. Sci. (USA) 1996;93:5281.

Gardner and Jack (Nucl. Acids Res. 1999; 27:2545) describe mutations in Vent DNA polymerase that enhance the incorporation of ribo-, 2' and 3'deoxyribo- and 2' 3' -dideoxy-ribonucleotides. The two individual mutations in Vent polymerase, Y412V and A488L, enhanced the relative activity of the enzyme with the nucleotide ATP. In addition, other substitutions at Y412 and A488 also increased ribonucleotide incorporation, though to a lesser degree. It was concluded that the bulk of the amino acid side chain at residue 412 acts as a "steric gate" to block access of the 2'-hydroxyl of the ribonucleotide sugar to the binding site. However, the rate enhancement with cordycepin (3'-deoxy adenosine triphosphate) was only 2-fold, suggesting that the Y412V polymerase variant was also sensitive to the loss of the 3' sugar hydroxyl. For residue A488, the change in activity is less easily rationalized. A488 is predicted to point away from the nucleotide binding site; here the enhancement in activity was explained through a change to the activation energy required for the enzymatic reaction. These mutations in Vent correspond to Y409 and A485 in 9°N polymerase.

The universality of the A488L mutation has been confirmed by homologous mutations in the following hyperthermophilic polymerases:
A486Y variant of Pfu DNA polymerase (Evans et al., Nucl. Acids Res. 2000; 28:1059). A series of random mutations were introduced into the polymerase gene and variants were identified that had improved incorporation of ddNTPs. The A486Y mutation improved the ratio of ddNTP/dNTP in sequencing ladders by 150-fold compared to wild type. However, mutation of Y410 to A or F produced a variant that resulted in an inferior sequencing ladder compared to the wild type enzyme. For further information, reference is made to International Publication No. WO 01/38546.

A485L variant of 9°N DNA polymerase (Gardner and Jack, Nucl. Acids Res. 2002; 30:605). This study demonstrated that the mutation of alanine to leucine at amino acid 485 enhanced the incorporation of nucleotide analogues that lack a 3' sugar hydroxyl moiety (acyNTPs and dideoxyNTPs).

A485T variant of Tsp JDF-3 DNA polymerase (Arezi et al., J. Mol. Biol. 2002;322:719). In this paper, random mutations were introduced into the JDF-3 polymerase from which variants were identified that had enhanced incorporation of ddNTPs. Individually, two mutations, A485T and P410L, improved ddNTP uptake compared to the wild type enzyme. In combination, these mutations had an additive effect and improved ddNTP incorporation by 250-fold. This paper demonstrates that the simultaneous mutation of two regions of a DNA polymerase can have additive effects on nucleotide analogue incorporation. In addition, this report demonstrates that P410, which lies adjacent to Y409 described above, also plays a role in the discrimination of nucleotide sugar analogues.

WO 01/23411 describes the use of the A488L variant of Vent in the incorporation of dideoxynucleotides and acyclonucleotides into DNA. The application also covers methods of sequencing that employ these nucleotide analogues and variants of 9°N DNA polymerase that are mutated at residue 485.

WO 2005/024010 A1 also relates to the modification of the motif A region and to the 9°N DNA polymerase. Yet, the modifications today still do not show sufficiently high incorporation rates of modified nucleotides. It would therefore be beneficial to have enzymes that have such high incorporation rates of modified nucleotides.

### SUMMARY OF THE INVENTION

To improve the efficiency of certain DNA sequencing methods, the inventors have analyzed whether DNA polymerases could be modified to produce improved rates of incorporation of such 3' substituted nucleotide analogues.

The invention relates to a family B polymerase enzyme comprising the following mutations, a mutation in the motif A region, wherein a threonine is the second amino acid of the motif A region, wherein the motif A region is homologous to amino acids 408 to 410 in 9°N, a mutation leading to a reduction or elimination of the 3'-5' exonuclease activity if present in said family B polymerase, wherein said polymerase additionally comprises one or both of the following mutations in the motif A region, L408Y or L408F and/or P410S or P410G.

The invention also relates to the use of a modified polymerase in DNA sequencing and a kit comprising such an enzyme.

Herein, "incorporation" means joining of the modified nucleotide to the free 3' hydroxyl group of a second nucleotide via formation of a phosphodiester linkage with the 5' phosphate group of the modified nucleotide. The second nucleotide to which the modified nucleotide is joined will typically occur at the 3' end of a polynucleotide chain.

Herein, "modified nucleotides" and "nucleotide analogues" when used in the context of this invention refer to nucleotides which have been modified at the 3' sugar hydroxyl such that the substituent is larger in size than the naturally occurring 3' hydroxyl group. These terms may be used interchangeably.

Herein, the term "large 3' substituent(s)" refers to a substituent group at the 3' sugar hydroxyl, which is larger in size than the naturally occurring 3' hydroxyl group.

Herein, "improved" incorporation is defined to include an increase in the efficiency and/or observed rate of incorporation of at least one modified nucleotide, compared to a control polymerase enzyme. However, the invention is not limited just to improvements in absolute rate of incorporation of the modified nucleotides. As shown below the polymerases also incorporate other modifications and so called dark nucleotides, hence, "improved incorporation" is to be interpreted accordingly as also encompassing improvements in any of these other properties, with or without an increase in the rate of incorporation. The "improvement" does not need to be constant over all cycles. Herein, "improvement" may be the ability to incorporate the modified nucleotides at low temperatures and/or over a wider temperature range than the control enzyme. Herein, "improvement" may be the ability to incorporate the modified nucleotides when using a lower concentration of the modified nucleotides as substrate. Preferably, the altered polymerase should exhibit detectable incorporation of the modified nucleotide when working at a substrate concentration in the nanomolar range.

Herein, "altered polymerase enzyme" means that the polymerase has at least one amino acid change compared to the control polymerase enzyme. In general, this change will comprise the substitution of at least one amino acid for another. In certain instances, these changes will be conservative changes to maintain the overall charge distribution of the protein. However, the invention is not only limited to conservative substitutions. Non-conservative substitutions are also envisaged in the present invention. Moreover, it is within the contemplation of the present invention that the modification in the polymerase sequence may be a deletion or addition of one or more amino acids from or to the protein, provided that the polymerase has improved activity with respect to the incorporation of nucleotides modified at the 3' sugar hydroxyl such that the substituent is larger in size than the naturally occurring 3' hydroxyl group as compared to a control polymerase enzyme.

Herein, "nucleotide" is defined to include both nucleotides and nucleosides. Nucleosides, as for nucleotides, comprise a purine or pyrimidine base linked glycosidically to ribose or deoxyribose, but they lack the phosphate residues, which would make them a nucleotide. Synthetic and naturally occurring nucleotides, prior to their modification at the 3' sugar hydroxyl, are included within the definition.

Herein and throughout the specification, mutations within the amino acid sequence of a polymerase are written in the following form: (i) single letter amino acid as found in wild type polymerase, (ii) position of the change in the amino acid sequence of the polymerase and (iii) single letter amino acid as found in the altered polymerase. So, mutation of a tyrosine residue in the wild type polymerase to a valine residue in the altered polymerase at position 409 of the amino acid sequence would be written as Y409V. This is standard procedure in molecular biology.

### DETAILED DESCRIPTION OF THE INVENTION

The sheer increase in rates of incorporation of the modified analogues that have been achieved with polymerases of the invention is unexpected. The examples show that even existing polymerases with mutations do not exhibit these high incorporation rates.

The invention relates to a family B polymerase enzyme comprising the following mutations, a mutation in the motif A region, wherein, in the native form, a tyrosine is the second amino acid of the motif A region, wherein the motif A region is homologous to amino acids 408 to 410 in 9°N, a mutation leading to a reduction or elimination of the 3'-5' exonuclease activity if present in said family B polymerase, wherein said polymerase additionally comprises one or both of the following mutations in the motif A region, L408Y or L408F and/or P410S or P410G. With reference to these additional modifications, they may be interchanged, hence the enzyme may have, e.g. L408Y and P410S, L408Y and P410G, L408F and P410S or L408F and P410G. Preferable these are in addition to the mutation, wherein a threonine is the second amino acid of the motif A region, wherein the motif A region is homologous to amino acids 408 to 410 in 9°N.

The altered polymerase will generally and preferably be an "isolated" or "purified" polypeptide. By "isolated polypeptide" a polypeptide that is essentially free from contaminating cellular components is meant, such as carbohydrates, lipids, nucleic acids or other proteinaceous impurities which may be associated with the polypeptide in nature. The inventors have used a his-tag for purification, but other means may be used. Preferably, at least the altered polymerase may be a "recombinant" polypeptide.

The altered polymerase according to the invention may be a family B type DNA polymerase, or a mutant or variant thereof. Family B DNA polymerases include numerous archaeal DNA polymerase, human DNA polymerase α and T4, RB69 and φ29 phage DNA polymerases. Family A polymerases include polymerases such as Taq and T7 DNA polymerase. In one embodiment, the polymerase is selected from any family B archaeal DNA polymerase, human DNA polymerase α or T4, RB69 and φ29 phage DNA polymerases.

Preferably, the polymerase is selected from the group consisting of 9°N polymerase having the mutation Y409T, Vent polymerase having the mutation Y412T, Pfu polymerase having the mutation Y410T, JDF-3 polymerase having the mutation Y409T and Taq polymerase having the mutation E615T.

Ideally, the polymerase comprises one or more mutations, corresponding to the following mutations of 9°N polymerase D215A, D315A, D141A and/or E143A or combinations thereof. In a preferred embodiment, the polymerase comprises mutation corresponding to 9°N D141A and E143A or D215A and D315A.

Preferably, the modified polymerase additionally comprises a mutation corresponding to A485L in 9°N polymerase. This mutation corresponds to A488L in Vent.

Several other groups have published on this mutation.

A486Y variant of Pfu DNA polymerase (Evans et al., Nucl. Acids Res. 2000; 28:1059). A series of random mutations was introduced into the polymerase gene and variants were identified that had improved incorporation of ddNTPs. The A486Y mutation improved the ratio of ddNTP/dNTP in sequencing ladders by 150-fold compared to wild type. However, mutation of Y410 to A or F produced a variant that resulted in an inferior sequencing ladder compared to the wild type enzyme; see also WO 01/38546.

A485L variant of 9°N DNA polymerase (Gardner and Jack, Nucl. Acids Res. 2002; 30:605). This study demonstrated that the mutation of alanine to leucine at amino acid 485 enhanced the incorporation of nucleotide analogues that lack a 3' sugar hydroxyl moiety (acyNTPs and dideoxyNTPs).

A485T variant of Tsp JDF-3 DNA polymerase (Arezi et al., J. Mol. Biol. 2002;322:719). In this paper, random mutations were introduced into the JDF-3 polymerase from which variants were identified that had enhanced incorporation of ddNTPs.

WO 01/23411 describes the use of the A488L variant of Vent in the incorporation of dideoxynucleotides and acyclonucleotides into DNA. The application also covers methods of sequencing that employ these nucleotide analogues and variants of 9°N DNA polymerase that are mutated at residue 485.

Ideally and preferably, the enzyme has the following set of mutations, (i) D215A, D315A, L408Y, Y409T, P410S and A485L or, (ii) D215A, D315A, L408F, Y409T, P410G and A485L.

Preferably, the polymerase is 3'-5' exonuclease minus and has the following mutations: L408Y, Y409T, P410S and A485L or, L408F, Y409T, P410G and A485L

The invention relates to a polymerase with the mutations shown herein which exhibits an increased rate of incorporation of nucleotides, which have been modified at the 3' sugar hydroxyl such that the substituent is larger in size than the naturally occurring 3' hydroxyl group, compared to the control polymerase.

Such nucleotides are disclosed in WO2004018497 A2. Here, a modified nucleotide molecule comprising a purine or pyrimidine base and a ribose or deoxyribose sugar moiety having a removable 3'-OH blocking group covalently attached thereto, such that the 3' carbon atom has attached a group of the structure: -O-Z is disclosed, wherein Z is any of -C(R')₂-N(R")₂'C(R')₂-N(H)R", and -C(R')₂-N₃, wherein each R" is or is part of a removable protecting group; each R' is independently a hydrogen atom, an alkyl, substituted alkyl, arylalkyl, alkenyl, alkynyl, aryl, heteroaryl, heterocyclic, acyl, cyano, alkoxy, aryloxy, heteroaryloxy or amido group, or a detectable label attached through a linking group; or (R')₂ represents an alkylidene group of formula =C(R‴)₂ wherein each R'" may be the same or different and is selected from the group comprising hydrogen and halogen atoms and alkyl groups; and wherein said molecule may be reacted to yield an intermediate in which each R" is exchanged for H, which intermediate dissociates under aqueous conditions to afford a molecule with a free 3'-OH.

The invention also relates to a nucleic acid molecule encoding a polymerase according to the invention, as well as an expression vector comprising said nucleic acid molecule.

The invention also relates to a method for incorporating nucleotides which have been modified at the 3' sugar hydroxyl such that the substituent is larger in size than the naturally occurring 3' hydroxyl group into DNA comprising the following substances (i) a polymerase according to any one of claims 1 to 7, (ii) template DNA, (iii) one or more nucleotides, which have been modified at the 3' sugar hydroxyl such that the substituent is larger in size than the naturally occurring 3' hydroxyl group.

The invention also relates to the use of a polymerase according to the invention in methods such as nucleic acid labeling or sequencing. The polymerases of the present invention are useful in a variety of techniques requiring incorporation of a nucleotide into a polynucleotide, which include sequencing reactions, polynucleotide synthesis, nucleic acid amplification, nucleic acid hybridization assays, single nucleotide polymorphism studies, and other such techniques. All such uses and methods utilizing the modified polymerases of the invention are included within the scope of the present invention.

In sequencing, the use of nucleotides bearing a 3' block allows successive nucleotides to be incorporated into a polynucleotide chain in a controlled manner. After each nucleotide addition the presence of the 3' block prevents incorporation of a further nucleotide into the chain. Once the nature of the incorporated nucleotide has been determined, the block may be removed, leaving a free 3' hydroxyl group for addition of the next nucleotide. Sequencing by synthesis of DNA ideally requires the controlled (i.e. one at a time) incorporation of the correct complementary nucleotide opposite the oligonucleotide being sequenced. This allows for accurate sequencing by adding nucleotides in multiple cycles as each nucleotide residue is sequenced one at a time, thus preventing an uncontrolled series of occurring incorporations. The incorporated nucleotide is read using an appropriate label attached thereto before removal of the label moiety and the subsequent next round of sequencing. In order to ensure only a single incorporation occurs, a structural modification ("blocking group") of the sequencing nucleotides is required to ensure a single nucleotide incorporation but which then prevents any further nucleotide incorporation into the polynucleotide chain. The blocking group must then be removable, under reaction conditions, which do not interfere with the integrity of the DNA being sequenced. The sequencing cycle can then continue with the incorporation of the next blocked, labelled nucleotide. In order to be of practical use, the entire process should consist of high yielding, highly specific chemical and enzymatic steps to facilitate multiple cycles of sequencing. To be useful in DNA sequencing, nucleotide, and more usually nucleotide triphosphates, generally require a 3'-OH blocking group so as to prevent the polymerase used to incorporate it into a polynucleotide chain from continuing to replicate once the base on the nucleotide is added. The DNA template for a sequencing reaction will typically comprise a double-stranded region having a free 3' hydroxyl group which serves as a primer or initiation point for the addition of further nucleotides in the sequencing reaction. The region of the DNA template to be sequenced will overhang this free 3' hydroxyl group on the complementary strand. The primer bearing the free 3' hydroxyl group may be added as a separate component (e.g. a short oligonucleotide) which hybridizes to a region of the template to be sequenced. Alternatively, the primer and the template strand to be sequenced may each form part of a partially self-complementary nucleic acid strand capable of forming an intramolecular duplex, such as for example a hairpin loop structure. Nucleotides are added successively to the free 3' hydroxyl group, resulting in synthesis of a polynucleotide chain in the 5' to 3' direction. After each nucleotide addition, the nature of the base, which has been added, will be determined, thus providing sequence information for the DNA template.

Such DNA sequencing may be possible if the modified nucleotides can act as chain terminators. Once the modified nucleotide has been incorporated into the growing polynucleotide chain complementary to the region of the template being sequenced there is no free 3'-OH group available to direct further sequence extension and therefore the polymerase cannot add further nucleotides. Once the nature of the base incorporated into the growing chain has been determined, the 3' block may be removed to allow addition of the next successive nucleotide. By ordering the products derived using these modified nucleotides it is possible to deduce the DNA sequence of the DNA template. Such reactions can be done in a single experiment if each of the modified nucleotides has attached a different label, known to correspond to the particular base, to facilitate discrimination between the bases added at each incorporation step. Alternatively, a separate reaction may be carried out containing each of the modified nucleotides separately.

In a preferred embodiment, the modified nucleotides carry a label to facilitate their detection. Preferably, this is a fluorescent label. Each nucleotide type may carry a different fluorescent label. However, the detectable label need not be a fluorescent label. Any label can be used which allows the detection of the incorporation of the nucleotide into the DNA sequence.

One method for detecting the fluorescently labelled nucleotides, suitable for use in the second and third aspects of the invention, comprises using laser light of a wavelength specific for the labelled nucleotides or the use of other suitable sources of illumination.

In one embodiment the fluorescence from the label on the nucleotide may be detected by a CCD camera.

If the DNA templates are immobilised on a surface they may preferably be immobilised on a surface to form a high density array. Most preferably, and in accordance with the technology developed by the applicants for the present invention, the high density array comprises a single molecule array, wherein there is a single DNA molecule at each discrete site that is detectable on the array. Single-molecule arrays comprised of nucleic acid molecules that are individually resolvable by optical means and the use of such arrays in sequencing are described, for example, in WO 00/06770, the contents of which are incorporated herein by reference. Single molecule arrays comprised of individually resolvable nucleic acid molecules including a hairpin loop structure are described in WO 01/57248, the contents of which are also incorporated herein by reference. The polymerases of the invention are suitable for use in conjunction with single molecule arrays prepared according to the disclosures of WO 00/06770 of WO 01/57248. However, it is to be understood that the scope of the invention is not intended to be limited to the use of the polymerases in connection with single molecule arrays. Single molecule array-based sequencing methods may work by adding fluorescently labelled modified nucleotides and an altered polymerase to the single molecule array. Complementary nucleotides would base-pair to the first base of each nucleotide fragment and would be added to the primer in a reaction catalysed by the improved polymerase enzyme. Remaining free nucleotides would be removed. Then, laser light of a specific wavelength for each modified nucleotide would excite the appropriate label on the incorporated modified nucleotides, leading to the fluorescence of the label. This fluorescence could be detected by a suitable CCD camera that can scan the entire array to identify the incorporated modified nucleotides on each fragment. Thus, millions of sites could potentially be detected in parallel. Fluorescence could then be removed. The identity of the incorporated modified nucleotide would reveal the identity of the base in the sample sequence to which it is paired. The cycle of incorporation, detection and identification would then be repeated approximately 25 times to determine the first 25 bases in each oligonucleotide fragment attached to the array, which is detectable. Thus, by simultaneously sequencing all molecules on the array, which are detectable, the first 25 bases for the hundreds of millions of oligonucleotide fragments attached in single copy to the array could be determined. Obviously, the invention is not limited to sequencing 25 bases. Many more or less bases could be sequenced depending on the level of detail of sequence information required and the complexity of the array. Using a suitable bioinformatics program the generated sequences could be aligned and compared to specific reference sequences. This would allow determination of any number of known and unknown genetic variations such as single nucleotide polymorphisms (SNPs) for example. The utility of the altered polymerases of the invention is not limited to sequencing applications using single-molecule arrays. The polymerases may be used in conjunction with any type of array-based (and particularly any high density array-based) sequencing technology requiring the use of a polymerase to incorporate nucleotides into a polynucleotide chain, and in particular any array-based sequencing technology which relies on the incorporation of modified nucleotides having large 3' substituents (larger than natural hydroxyl group), such as 3' blocking groups. The polymerases of the invention may be used for nucleic acid sequencing on essentially any type of array formed by immobilisation of nucleic acid molecules on a solid support. In addition to single molecule arrays suitable arrays may include, for example, multi-polynucleotide or clustered arrays in which distinct regions on the array comprise multiple copies of one individual polynucleotide molecule or even multiple copies of a small-number of different polynucleotide molecules (e.g. multiple copies of two complementary nucleic acid strands). In particular, the polymerases of the invention may be utilised in the nucleic acid sequencing method described in WO 98/44152, the contents of which are incorporated herein by reference. This International application describes a method of parallel sequencing of multiple templates located at distinct locations on a solid support. The method relies on incorporation of labelled nucleotides into a polynucleotide chain. The polymerases of the invention may be used in the method described in International application WO 00/18957, the contents of which are incorporated herein by reference. This application describes a method of solid-phase nucleic acid amplification and sequencing in which a large number of distinct nucleic acid molecules are arrayed and amplified simultaneously at high density via formation of nucleic acid colonies and the nucleic acid colonies are subsequently sequenced. The altered polymerases of the invention may be utilised in the sequencing step of this method. Multi-polynucleotide or clustered arrays of nucleic acid molecules may be produced using techniques generally known in the art. By way of example, WO 98/44151 and WO 00/18957 both describe methods of nucleic acid amplification, which allow amplification products to be immobilised on a solid support in order to form arrays comprised of clusters or "colonies" of immobilised nucleic acid molecules. The contents of WO 98/44151 and WO 00/18957 relating to the preparation of clustered arrays and use of such arrays as templates for nucleic acid sequencing are incorporated herein by reference. The nucleic acid molecules present on the clustered arrays prepared according to these methods are suitable templates for sequencing using the polymerases of the invention. However, the invention is not intended to use of the polymerases in sequencing reactions carried out on clustered arrays prepared according to these specific methods. The polymerases of the invention may further be used in methods of fluorescent in situ sequencing, such as that described by Mitra et al. (Analytical Biochemistry 2003; 320:55).

Additionally, in another aspect, the invention provides a kit, comprising: (a) the polymerase according to the invention, and optionally, a plurality of different individual nucleotides of the invention and/or packaging materials therefor.

### EXAMPLES

Various 9°N mutants were generated. Surprisingly two mutants were found in this approach, which showed exceptionally good incorporation rates for modified nucleotides. The amino acid sequence of the polymerases comprise following substitution mutations:
PLA159 [SEQ ID NO 2]:
   D215A / D315A/ L408Y / Y409T / P410S / A485L / C-terminal His-tag
PLA163 [SEQ ID NO 4]:
   D215A / D315A/ L408F / Y409T / P410G / A485L / C-terminal His-tag

**Table 1 below depicts the new sequencing by synthesis polymerases developed in this study. Columns 2-5 describes alterations within their amino acid sequence compared to the formerly commercially available 9°N polymerase variant Therminator III polymerase (also referred to as T3 herein):**

| Internal designation | AA combination in LYP Motif / motif A | A485L | D215A/D315A | D141A/E143A |
|---|---|---|---|---|
| PLA159 | YTS | + | + | - |
| PLA163 | FTG | + | + | - |

### Mutant purification

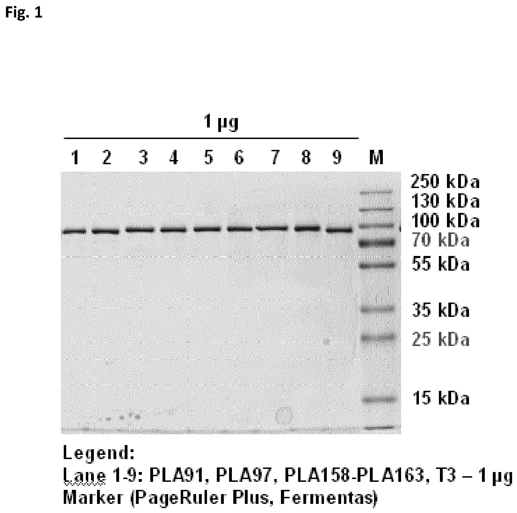

The his-tagged mutant polymerases were purified in preparative scale via Ni-NTA-superflow column. The mutants were 99% pure according to SDS-Gel analysis (Fig. 1).

### Single base incorporation assay (CE-assay)

To measure the ability of different polymerase mutants to incorporate labelled nucleotides (= nucleotide + blocking group + linker-fluorophore group) a single base incorporation assay was performed.

A labeled universal primer and four non labeled templates, each for testing one of the four labeled nucleotides or one of the four dark nucleotides (= nucleotide + blocking group), were used for the extension reaction. Hybridized template primer pairs of 24 base pairs and 18 base pairs, respectively, were used at a ratio of 2:1. The reaction was started either by adding the enzyme or the nucleotide to the reaction. The one base extension at the 3' end of the labeled primer was measured in defined intervals over a timeframe of 120 seconds and analyzed with a capillary electrophoresis analyser. The amounts of extended and non-extended primer were calculated via the fluorescence peak areas of the electropherograms and the ratio was plotted against time.

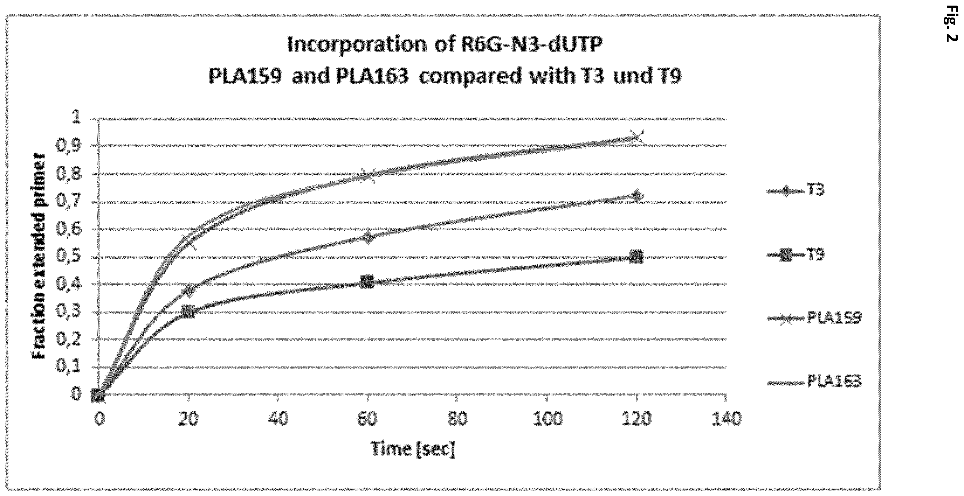

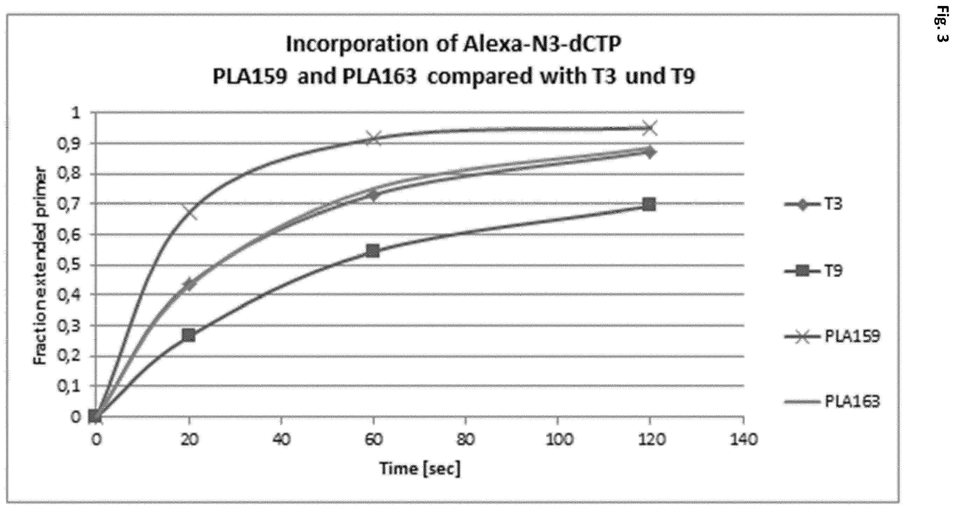

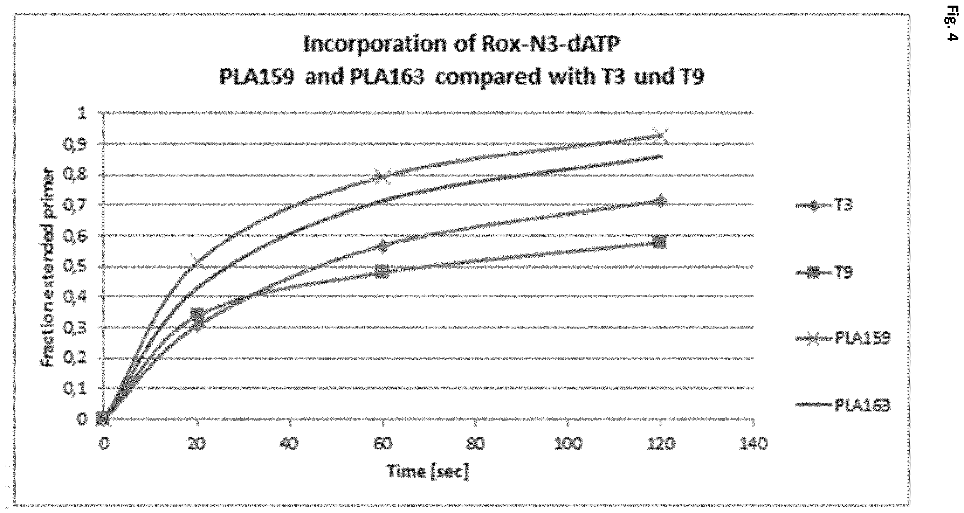

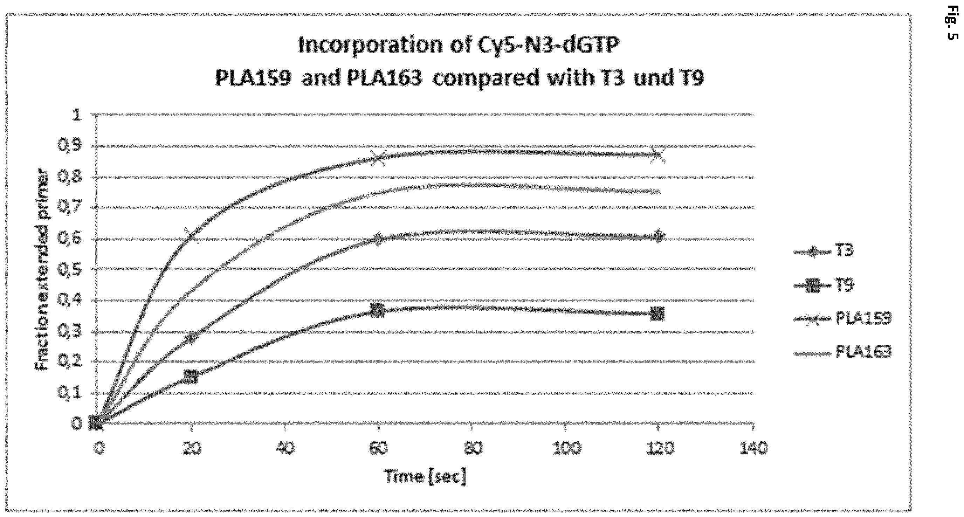

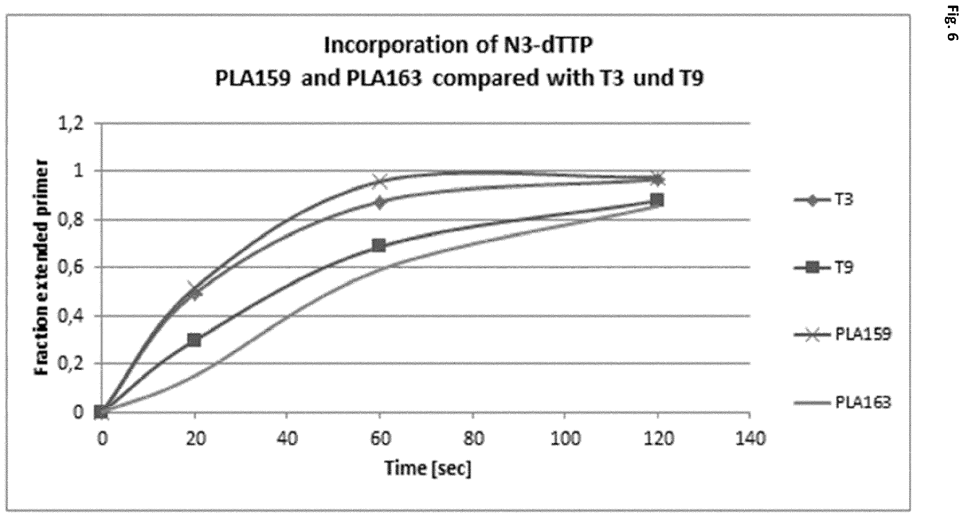

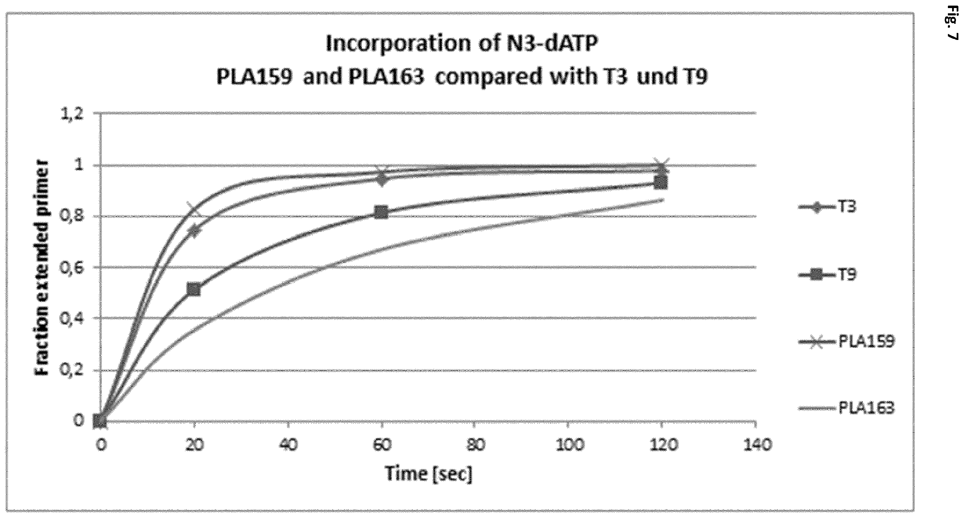

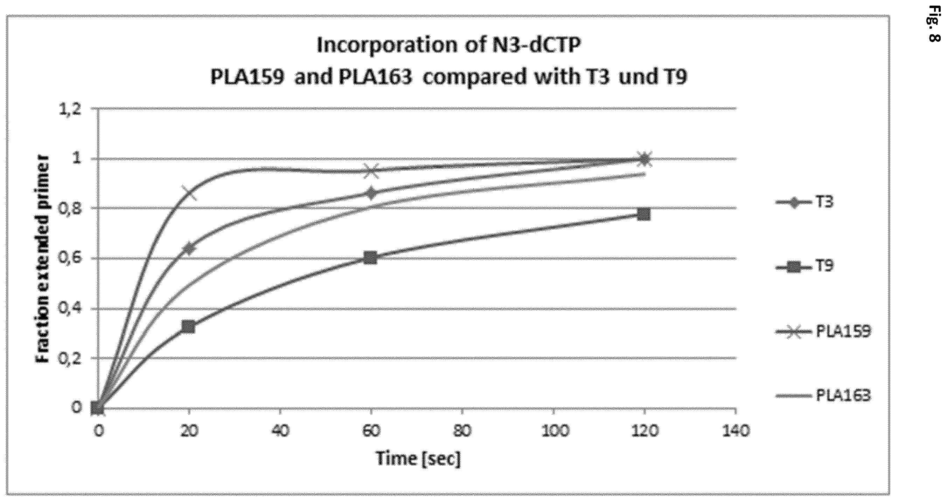

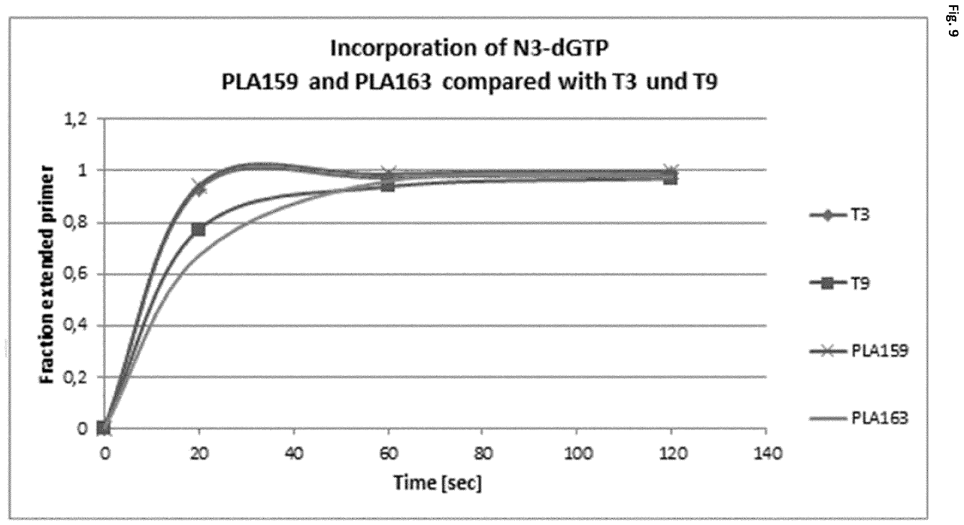

Detailed data on the incorporation of labeled and dark nucleotides of PLA159 and PLA163 compared to T3 and T9 is shown in Fig 2 to 9.

All polymerases showed good incorporation performance with labeled nucleotides. The highest incorporation rates with all nucleotides had PLA159. They were even higher than the T3 reference. The new polymerase mutants generally performed much better in the CE-assay.

### Exonuclease activity without addition of matching nucleotides

For the measurement of exonuclease activity 12.5 nM of FAM-labeled primer was annealed to 25 nM template DNA. Polymerase was added to the template / primer mix after the annealing step. The reaction mix was incubated for 60 minutes at 65°C without addition of nucleotides. After the incubation period the reaction products were analyzed by capillary electrophoresis. De-graded oligonucleotide and non- de-graded oligonucleotide differed in size (peak size of non-degraded oligonucleotide: 7-7.2; de-graded oligonucleotide: <7). With the help of the fluorescence peak areas of the different sized peaks, the amount of degraded primer was calculated.

Like T3 and T9 both mutant polymerases PLA159 and PLA163 showed no detectable exonuclease activity as no peaks smaller than the FAM primer peak could be detected.

**Table 1: Exonuclease activity [%] of T3, T9, PLA159, PLA163 and a polymerase that exhibit exonulease activity (positive control). PLA159 and PLA163 showed no exonuclease activity.**

| Polymerase | Undigested primer | Digested primer |
|---|---|---|
| T3 | 100% | nd |
| T9 | 100% | nd |
| PLA159 | 100% | nd |
| PLA163 | 100% | nd |
| Positive control | 51% | 49% |

### Misincorporation with matching nucleotides

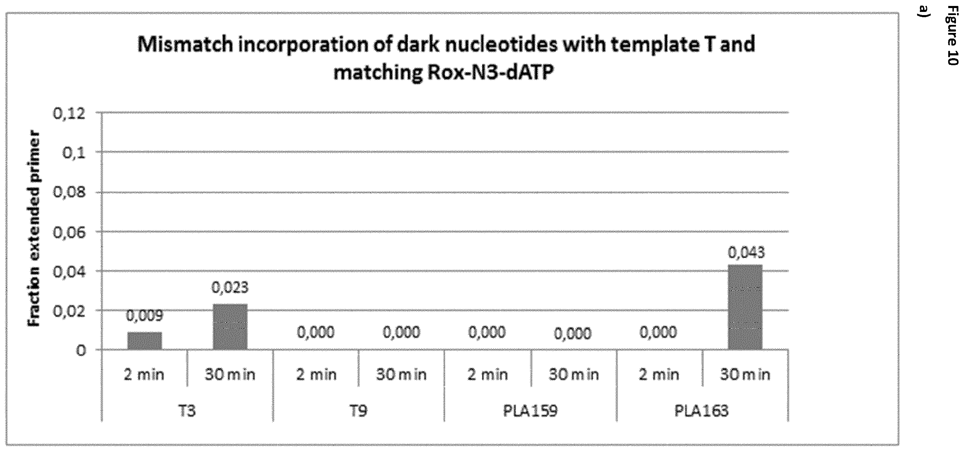

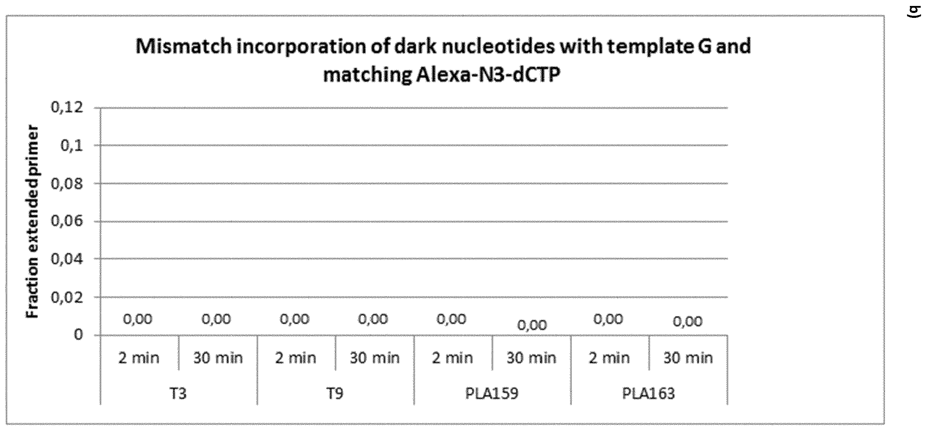

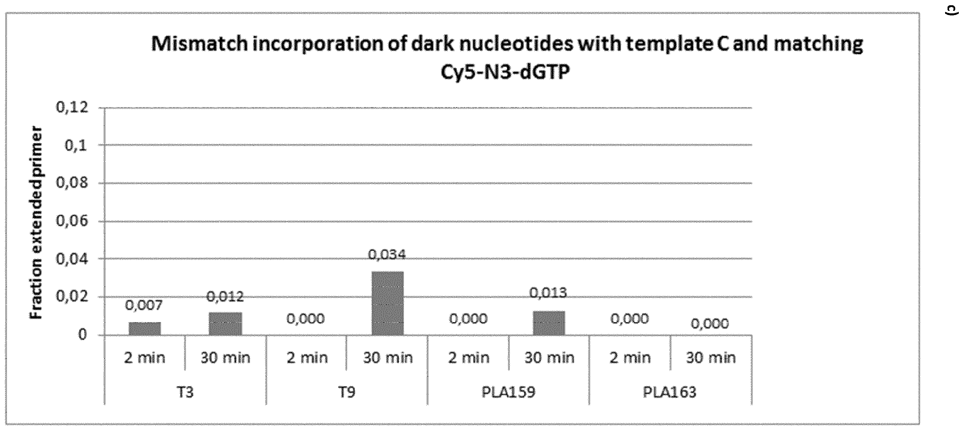

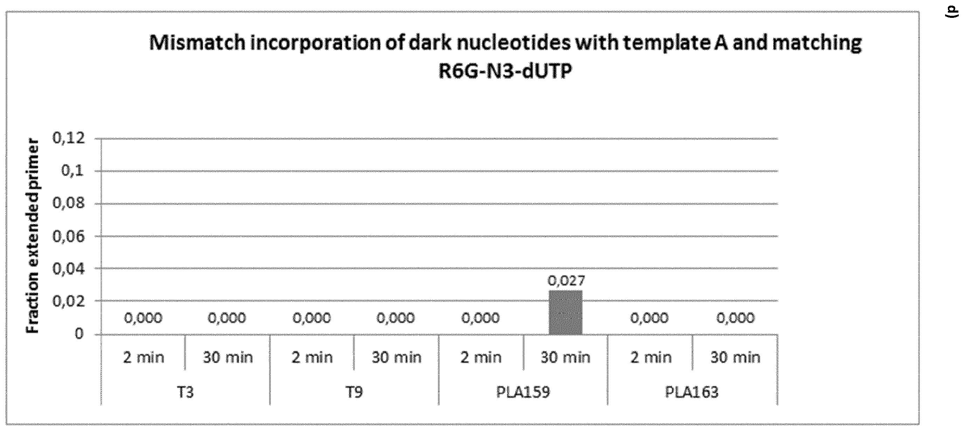

In the experimental set-up the enzyme was incubated with a template-primer mix and 4 nucleotides (three non-matching nucleotides and one matching nucleotide). The incorporation was measured after 2 minutes, the timeframe given for nucleotide incorporation during the extension phase of a GeneReader run, and 30 minutes (Fig. 10). PLA159 and PLA163 were compared with T3 and T9. As mismatch incorporation is likely to hamper the sequencing performance polymerases with little to non-mismatch incorporation should be chosen for sequencing.

PLA159 and PLA163 only exhibited mismatch incorporation after 30 minutes and the misincorporation rate was mostly comparable to the T3 and T9 references.

PLA159 showed insignificant mismatch incorporation after 30 minutes on template A and template C (0,027 and 0,013 respectively). Mismatch incorporation for PLA163 was only measured on template T (0,043).

The positive controls T3 and T9 also showed low level mismatch incorporation. For T3 mismatch incorporation was already detected after 2 minutes incubation on template T and template C (0,007 and 0,009). The rate slightly increased after 30 minutes (0,012 and 0,023). Mismatch incorporation for T9 was only detected after 30 minutes incubation on template C (0,034). As both polymerases are used as sequencing polymerase these low level misincorporations does not seem to have an impact on the sequencing reaction. Therefore, it is assumed that the measured low level misincorporation of PLA159 and PLA163 do not hamper any sequencing reaction. Potential mismatch incorporation of PLA159 and PLA163 are insignificant and are unlikely to have a negative effect on the sequencing reaction.

Both mutants showed excellent incorporation rates for labeled and dark nucleotides and can be used for applications using modified nucleotides like next generation sequencing. As both exonuclease activity as well as mismatch incorporation may have an influence on an enzyme's applicability for sequencing reactions, we evaluated both relative to T3 and T9. Our data indicates that PLA159 and PLA163 do not show elevated mismatch incorporation or exonuclease activity.

In view of the performance data and considering all demands for a new sequencing by synthesis polymerase (activity, mismatch incorporation, exonuclease activity) both polymerases, PLA159 and PLA163, are better than those known in the art.

### Comparision of several wild-type polymerases and reverse transcriptases with T3

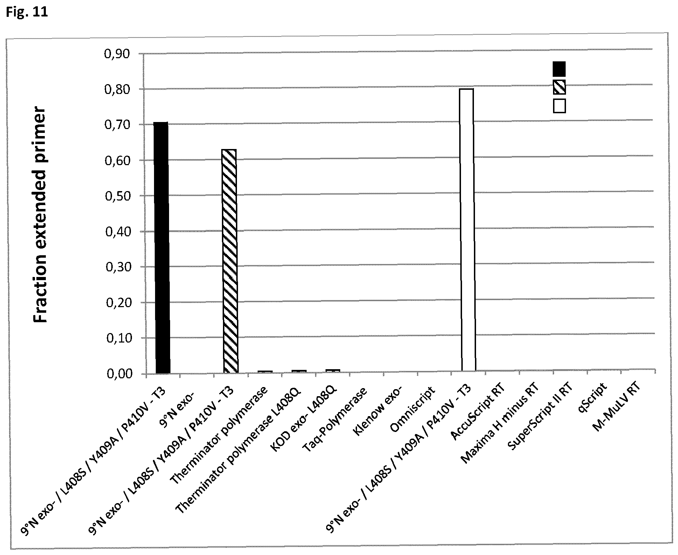

The incorporation activity of T3 for labeled nucleotides was compared with different polymerases and reverse transcriptases. For the comparison of T3 (9°N exo⁻ / L408S / Y409A / P410V) with an exo⁻ variant of the 9°N wildtype polymerase Rox-N3-dATP was used (Figure 11, black bars). The T3 polymerase was also compared to other 9°N exo⁻ mutants that carried single (Therminator polymerase; mutation A485L) or double mutations (Therminator polymerase L408Q) as well as to a KOD exo⁻ mutant with a single mutation (L408Q), wildtype Taq-polymerse, Omniscript from Qiagen and to an exo-Klenow polymerase sold by Enzymatics. For the comparison of those enzymes versus T3 polymerase R6G-N3-dUTP was used (black / white bars). Additionally, various commercially available RT enzymes were compared with T3 using Cy5-N3-dGTP (white bars).

All tested enzymes showed no incorporation activity with the modified nucleotides used in the respective experiments. Thus, those mutations alone do not boost the incorporation of the tested modified nucleotides.

Conclusion: Wildtype enzymes like Taq-polymerase, Omniscript, other reverse transcriptases, exo⁻ variants of 9°N or KOD polymerases with the single mutation L408Q do not show detectable incorporation activity for the tested labelled nucleotides.

### Assay conditions:

Polymerase: 1 µg/ml
Nucleotide concentration: 125 nM
Template: 25 nM
Primer: 12,5 nM
Incubation time: 2 min
Temperature: 65°C

**Polymerases Analyzed:**

| **Enzyme designation** | **Alternative Designation** | **Supplier** |
|---|---|---|
| 9°N exo⁻ / L408S / Y409A / P410V - T3 | T3 | NEB |
| 9°N exo⁻ | / | / |
| Therminator polymerase | / | NEB |
| Therminator polymerase L408Q | / | / |
| KOD exo⁻ L408Q | / | / |
| Taq-Polymerase | / | Qiagen |
| Klenow exo⁻ | / | Enzymatics |
| Omniscript | / | / |
| AccuScript RT | / | Agilent Technologies |
| Maxima H minus RT | / | Thermo Scientific |
| SuperScript II RT | / | Life Technologies |
| qScript | / | Quanta Biosciences |
| M-MuLV RT | / | NEB |

**Enzyme Sequences**

| | |
|---|---|
| DNA sequence of PLA159: | AGATCTATGAAACACCACCACCATCATCACATTTTGGACACGGACTACAT CACTGAAAACGGTAAGCCGGTTATTCGCGTGTTTAAGAAAGAAAATGGTG AGTTCAAGATCGAGTACGACCGTACCTTTGAACCGTACTTCTATGCGCTG CTGAAAGACGACAGCGCCATCGAAGATGTGAAGAAGGTTACCGCGAAACG TCATGGCACCGTTGTTAAAGTCAAGCGTGCAGAGAAGGTGCAGAAGAAGT TTCTGGGTCGTCCGATCGAGGTGTGGAAACTGTATTTCAACCATCCGCAG GACGTTCCTGCCATCCGTGACCGTATTCGCGCACATCCGGCGGTTGTGGA CATTTACGAATACGACATCCCTTTCGCAAAACGTTATCTGATTGATAAGG GCTTGATTCCAATGGAAGGTGATGAAGAACTGACCATGCTGGCGTTTGAT ATCGAAACCCTGTACCACGAGGGCGAAGAGTTCGGTACGGGCCCTATTTT GATGATTTCCTACGCCGACGGCAGCGAAGCGCGTGTGATTACCTGGAAAA AGATTGATCTGCCGTATGTCGACGTCGTGAGCACCGAAAAAGAGATGATC AAGCGCTTTCTGCGTGTTGTGCGTGAGAAGGATCCGGACGTCCTGATTAC GTACAATGGTGACAACTTTGCTTTTGCGTATTTGAAAAAGCGTAGCGAAG AGCTGGGTATCAAGTTTACCCTGGGTCGCGATGGTAGCGAGCCAAAGATC CAACGTATGGGTGACCGCTTCGCGGTTGAGGTTAAAGGCCGTATCCACTT CGACCTGTACCCGGTCATCCGTCGCACCATCAATCTGCCGACCTATACCC TGGAAGCGGTCTATGAGGCTGTGTTCGGCAAGCCGAAAGAAAAGGTTTAT GCCGAAGAGATTGCACAGGCGTGGGAGAGCGGCGAGGGTCTGGAGCGTGT GGCACGCTATAGCATGGAAGCTGCCAAAGTGACCTATGAGCTGGGCCGTG AATTCTTCCCGATGGAAGCGCAGCTGAGCCGTCTGATTGGCCAAAGCCTG TGGGATGTAAGCCGCTCTTCTACGGGCAACCTGGTCGAGTGGTTCCTGCT GCGCAAGGCGTACAAACGTAATGAGCTGGCACCGAACAAACCGGATGAGC GTGAACTGGCCCGTCGTCGTGGTGGTTATGCGGGTGGCTACGTGAAAGAG CCGGAGCGTGGCTTGTGGGATAACATTGTGTACCTGGACTTCCGCAGCTA TACTTCTAGCATCATCATCACGCATAACGTTAGCCCGGACACGTTGAACC GTGAAGGTTGTAAAGAGTACGACGTTGCCCCGGAAGTTGGTCACAAGTTT TGTAAAGACTTCCCGGGTTTCATCCCGAGCCTGCTGGGTGATCTGCTGGA AGAACGCCAAAAGATCAAACGCAAGATGAAAGCGACGGTTGATCCGTTGG AGAAAAAGCTGTTGGATTACCGCCAACGTCTGATTAAGATTCTGGCAAAT AGCTTTTACGGTTACTACGGTTATGCAAAAGCCCGCTGGTATTGCAAAGA GTGCGCGGAGTCCGTCACGGCTTGGGGCCGTGAGTATATCGAAATGGTTA TCCGCGAGCTGGAAGAGAAATTCGGCTTTAAGGTCCTGTACGCGGACACC GACGGCCTGCACGCAACCATCCCGGGTGCAGATGCGGAGACTGTTAAGAA GAAGGCGAAAGAGTTTCTGAAATACATCAATCCGAAATTGCCGGGTCTGC TGGAGCTGGAATATGAGGGCTTTTATGTCCGTGGTTTCTTCGTGACCAAA AAGAAATACGCGGTCATTGACGAAGAGGGTAAGATTACGACCCGCGGTTT GGAGATTGTCCGTCGTGACTGGTCGGAGATCGCTAAAGAAACCCAAGCAC GTGTACTGGAAGCGATTCTGAAACACGGTGACGTGGAAGAGGCAGTTCGC ATCGTTAAAGAGGTGACGGAGAAGCTGAGCAAATATGAGGTTCCGCCTGA AAAGCTGGTGATTCATGAACAGATCACGCGCGATCTGCGCGATTACAAAG CGACCGGTCCGCACGTTGCGGTCGCCAAGCGTCTGGCGGCTCGCGGTGTC AAGATTCGTCCGGGCACGGTTATCAGCTACATCGTGCTGAAGGGTTCCGG TCGTATTGGCGATCGTGCTATTCCGGCGGACGAATTCGATCCGACCAAAC ACCGTTACGATGCTGAGTACTACATTGAAAATCAGGTGCTGCCGGCAGTT GAACGTATCCTGAAAGCATTTGGTTATCGTAAAGAGGATCTGCGCTACCA AAAGACCAAACAGGTCGGCCTGGGCGCCTGGCTGAAAGTGAAAGGTAAGA AGTAATGAAAGCTT |
| A141D/A143E/ D215A/D315A/ L408Y/Y409T/ P410S/C223S/ | |
| A485L (SEQ ID NO 1) | |
| PLA159 | MKHHHHHHILDTDYITENGKPVIRVFKKENGEFKIEYDRTFEPYFYALLKD DSAIEDVKKVTAKRHGTVVKVKRAEKVQKKFLGRPIEVWKLYFNHPQDVPA IRDRIRAHPAVVDIYEYDIPFAKRYLIDKGLIPMEGDEELTMLAFDIETLY HEGEEFGTGPILMISYADGSEARVITWKKIDLPYVDVVSTEKEMIKRFLRV VREKDPDVLITYNGDNFAFAYLKKRSEELGIKFTLGRDGSEPKIQRMGDRF AVEVKGRIHFDLYPVIRRTINLPTYTLEAVYEAVFGKPKEKVYAEEIAQAW ESGEGLERVARYSMEAAKVTYELGREFFPMEAQLSRLIGQSLWDVSRSSTG NLVEWFLLRKAYKRNELAPNKPDERELARRRGGYAGGYVKEPERGLWDNIV YLDFRSYTSSIIITHNVSPDTLNREGCKEYDVAPEVGHKFCKDFPGFIPSL LGDLLEERQKIKRKMKATVDPLEKKLLDYRQRLIKILANSFYGYYGYAKAR WYCKECAESVTAWGREYIEMVIRELEEKFGFKVLYADTDGLHATIPGADAE TVKKKAKEFLKYINPKLPGLLELEYEGFYVRGFFVTKKKYAVIDEEGKITT RGLEIVRRDWSEIAKETQARVLEAILKHGDVEEAVRIVKEVTEKLSKYEVP PEKLVIHEQITRDLRDYKATGPHVAVAKRLAARGVKIRPGTVISYIVLKGS GRIGDRAIPADEFDPTKHRYDAEYYIENQVLPAVERILKAFGYRKEDLRYQ KTKQVGLGAWLKVKGKKStop |
| Proteinsequen z (SEQ ID NO 2) | |
| | |
| PLA163: | AGATCTATGAAACACCACCACCATCATCACATTTTGGACACGGACTACATC ACTGAAAACGGTAAGCCGGTTATTCGCGTGTTTAAGAAAGAAAATGGTGAG TTCAAGATCGAGTACGACCGTACCTTTGAACCGTACTTCTATGCGCTGCTG AAAGACGACAGCGCCATCGAAGATGTGAAGAAGGTTACCGCGAAACGTCAT GGCACCGTTGTTAAAGTCAAGCGTGCAGAGAAGGTGCAGAAGAAGTTTCTG GGTCGTCCGATCGAGGTGTGGAAACTGTATTTCAACCATCCGCAGGACGTT CCTGCCATCCGTGACCGTATTCGCGCACATCCGGCGGTTGTGGACATTTAC GAATACGACATCCCTTTCGCAAAACGTTATCTGATTGATAAGGGCTTGATT CCAATGGAAGGTGATGAAGAACTGACCATGCTGGCGTTTGATATCGAAACC CTGTACCACGAGGGCGAAGAGTTCGGTACGGCCCTATTTTGATGATTTCC TACGCCGACGGCAGCGAAGCGCGTGTGATTACCTGGAAAAAGATTGATCTG CCGTATGTCGACGTCGTGAGCACCGAAAAAGAGATGATCAAGCGCTTTCTG CGTGTTGTGCGTGAGAAGGATCCGGACGTCCTGATTACGTACAATGGTGAC AACTTTGCTTTTGCGTATTTGAAAAAGCGTAGCGAAGAGCTGGGTATCAAG TTTACCCTGGGTCGCGATGGTAGCGAGCCAAAGATCCAACGTATGGGTGAC CGCTTCGCGGTTGAGGTTAAAGGCCGTATCCACTTCGACCTGTACCCGGTC ATCCGTCGCACCATCAATCTGCCGACCTATACCCTGGAAGCGGTCTATGAG GCTGTGTTCGGCAAGCCGAAAGAAAAGGTTTATGCCGAAGAGATTGCACAG GCGTGGGAGAGCGGCGAGGGTCTGGAGCGTGTGGCACGCTATAGCATGGAA GCTGCCAAAGTGACCTATGAGCTGGGCCGTGAATTCTTCCCGATGGAAGCG CAGCTGAGCCGTCTGATTGGCCAAAGCCTGTGGGATGTAAGCCGCTCTTCT ACGGGCAACCTGGTCGAGTGGTTCCTGCTGCGCAAGGCGTACAAACGTAAT GAGCTGGCACCGAACAAACCGGATGAGCGTGAACTGGCCCGTCGTCGTGGT GGTTATGCGGGTGGCTACGTGAAAGAGCCGGAGCGTGGCTTGTGGGATAAC ATTGTGTACCTGGACTTCCGCAGCTTTACTGGAGCATCATCATCACGCAT AACGTTAGCCCGGACACGTTGAACCGTGAAGGTTGTAAAGAGTACGACGTT GCCCCGGAAGTTGGTCACAAGTTTTGTAAAGACTTCCCGGGTTTCATCCCG AGCCTGCTGGGTGATCTGCTGGAAGAACGCCAAAAGATCAAACGCAAGATG AAAGCGACGGTTGATCCGTTGGAGAAAAAGCTGTTGGATTACCGCCAACGT CTGATTAAGATTCTGGCAAATAGCTTTTACGGTTACTACGGTTATGCAAAA GCCCGCTGGTATTGCAAAGAGTGCGCGGAGTCCGTCACGGCTTGGGGCCGT GAGTATATCGAAATGGTTATCCGCGAGCTGGAAGAGAAATTCGGCTTTAAG GTCCTGTACGCGGACACCGACGGCCTGCACGCAACCATCCCGGGTGCAGAT GCGGAGACTGTTAAGAAGAAGGCGAAAGAGTTTCTGAAATACATCAATCCG |
| A141D/A143E/D 215A/D315A/L4 08F/Y409T/P41 0G/C223S/A485 L | |
| (SEQ ID NO 3) | |
| | AAATTGCCGGGTCTGCTGGAGCTGGAATATGAGGGCTTTATGTCCGTGGT TTCTTCGTGACCAAAAAGAAATACGCGGTCATTGACGAAGAGGGTAAGATT ACGACCCGCGGTTTGGAGATTGTCCGTCGTGACTGGTCGGAGATCGCTAAA GAAACCCAAGCACGTGTACTGGAAGCGATTCTGAAACACGGTGACGTGGAA GAGGCAGTTCGCATCGTTAAAGAGGTGACGGAGAAGCTGAGCAAATATGAG GTTCCGCCTGAAAAGCTGGTGATTCATGAACAGATCACGCGCGATCTGCGC GATTACAAAGCGACCGGTCCGCACGTTGCGGTCGCCAAGCGTCTGGCGGCT CGCGGTGTCAAGATTCGTCCGGGCACGGTTATCAGCTACATCGTGCTGAAG GGTTCCGGTCGTATTGGCGATCGTGCTATTCCGGCGGACGAATTCGATCCG ACCAAACACCGTTACGATGCTGAGTACTACATTGAAAATCAGGTGCTGCCG GCAGTTGAACGTATCCTGAAAGCATTTGGTTATCGTAAAGAGGATCTGCGC TACCAAAAGACCAAACAGGTCGGCCTGGGCGCCTGGCTGAAAGTGAAAGGT AAGAAGTAATGAAAGCTT |
| | |
| PLA163 | MKHHHHHHILDTDYITENGKPVIRVFKKENGEFKIEYDRTFEPYFYALLK DDSAIEDVKKVTAKRHGTVVKVKRAEKVQKKFLGRPIEVWKLYFNHPQDV PAIRDRIRAHPAVVDIYEYDIPFAKRYLIDKGLIPMEGDEELTMLAFDIE TLYHEGEEFGTGPILMISYADGSEARVITWKKIDLPYVDVVSTEKEMIKR FLRVVREKDPDVLITYNGDNFAFAYLKKRSEELGIKFTLGRDGSEPKIQR MGDRFAVEVKGRIHFDLYPVIRRTINLPTYTLEAVYEAVFGKPKEKVYAE EIAQAWESGEGLERVARYSMEAAKVTYELGREFFPMEAQLSRLIGQSLWD VSRSSTGNLVEWFLLRKAYKRNELAPNKPDERELARRRGGYAGGYVKEPE RGLWDNIVYLDFRSFTGSIIITHNVSPDTLNREGCKEYDVAPEVGHKFCK DFPGFIPSLLGDLLEERQKIKRKMKATVDPLEKKLLDYRQRLIKILANSF YGYYGYAKARWYCKECAESVTAWGREYIEMVIRELEEKFGFKVLYADTDG LHATIPGADAETVKKKAKEFLKYINPKLPGLLELEYEGFYVRGFFVTKKK YAVIDEEGKITTRGLEIVRRDWSEIAKETQARVLEAILKHGDVEEAVRIV KEVTEKLSKYEVPPEKLVIHEQITRDLRDYKATGPHVAVAKRLAARGVKI RPGTVISYIVLKGSGRIGDRAIPADEFDPTKHRYDAEYYIENQVLPAVER ILKAFGYRKEDLRYQKTKQVGLGAWLKVKGKKStop |
| Proteinsequenz (SEQ ID NO 4) | |

### FIGURE Legends

### Figure 1:

Gel analysis of PLA159 and PLA163 mutants compared to PLA91, PLA97 and Therminator III polymerse. PLA159 is shown in lane 4 and PLA163 is shown in lane 8.

### Figure 2:

CE-activity data (labeled nucleotides):
Performance of PLA159 and PLA163 in CE-assay compared with T3 and the modified NEB 9°N polymerase (referred to as T9 herein). In the experiment, 1 µg/ml enzyme and 125 nM R6G-N3-dUTP were used. PLA 159 and PLA163 showed higher incorporation rates with the labeled nucleotide R6G-N3-dUTP than T9 and T3.

### Figure 3:

Performance of PLA159 and PLA163 in CE-assay compared with T3 and T9. In the experiment, 1 µg/ml enzyme and 125 nM Alexa-N3-dCTP were used. PLA159 showed higher incorporation rates with the labeled nucleotide Alexa-N3-dCTP than T9 and T3. PLA163 showed higher incorporation rate than T9 and a similar incorporation rate to T3.

### Figure 4:

Performance of PLA159 and PLA163 in CE-assay compared with T3 and T9. In the experiment, 1 µg/ml enzyme and 125 nM ROX-N3-dATP were used. PLA 159 and PLA163 showed higher incorporation rates with the labeled nucleotide Rox-N3-dATP than T9 and T3.

### Figure 5:

Performance of PLA159 and PLA163 in CE-assay compared with T3 and T9. In the experiment, 1 µg/ml enzyme and 125 nM Cy5-N3-dGTP were used. PLA 159 and PLA163 showed higher incorporation rates with the labeled nucleotide Cy5-N3-dGTP than T9 and T3.

### Figure 6:

Performance of PLA159 and PLA163 in CE-assay compared with T3 and T9. In the experiment, 1 µg/ml enzyme and 125 nM N3-dTTP were used. PLA159 showed higher incorporation rates with the dark nucleotide N3-dTTP than T3.

### Figure 7:

Performance of PLA159 and PLA163 in CE-assay compared with T3 and T9. In the experiment, 1 µg/ml enzyme and 125 nM N3-dATP were used. PLA159 showed higher incorporation rates with the dark nucleotide N3-dATP than T9 and similar incorporation rates compared with T3.

### Figure 8:

Performance of PLA159 and PLA163 in CE-assay compared with T3 and T9. In the experiment, 1 µg/ml enzyme and 125 nM N3-dCTP were used. PLA159 showed a higher incorporation rate with the dark nucleotide N3-dCTP than T3. The incorporation rate of PLA163 was higher than T9 and similar to T3.

### Figure 9:

Performance of PLA159 and PLA163 in CE-assay compared with T3 and T9. In the experiment, 1 µg/ml enzyme and 125 nM N3-dGTP were used. PLA159 showed a similar incorporation rate with the dark nucleotide N3-dGTP than T3. The incorporation rate of PLA163 was comparable to T9. All polymerases showed high incorporation rates with N3-dGTP.

### Figure 10a-d:

Nucleotide misincorporation measurement of T3, T9, PLA159 and PLA163. The incorporation was determined for each template with the CE assay.

### Figure 11:

T3 activity compared to various polymerases and reverse transcriptases

## Claims

1. A family B polymerase enzyme comprising the following mutations, a mutation in the motif A region, wherein a threonine is the second amino acid of the motif A region, wherein the motif A region is homologous to amino acids 408 to 410 in 9°N, a mutation leading to a reduction or elimination of the 3'-5' exonuclease activity, wherein said polymerase additionally comprises one or both of the following mutations in the motif A region, L408Y or L408F and/or P410S or P410G.

2. The polymerase of claim 1 wherein the polymerase is selected from the group consisting of 9°N polymerase having the mutation Y409T, Vent polymerase having the mutation Y412T, Pfu polymerase having the mutation Y410T, JDF-3 polymerase having the mutation Y409T and Taq polymerase having the mutation E615T.

3. Polymerase according to claims 1 or 2, wherein the polymerase comprises one or more of the following mutations D215A and/or D315A.

4. Polymerase according to any of the claims 1 to 3, wherein the polymerase additionally comprises a mutation A485L.

5. Polymerase according to any of the preceding claims, wherein the enzyme has the following set of mutations, (i) D215A, D315A, L408Y, Y409T, P410S and A485L or, (ii) D215A, D315A, L408F, Y409T, P410G and A485L.

6. The polymerase according to any preceding claim which exhibits an increased rate of incorporation of nucleotides which have been modified at the 3' sugar hydroxyl such that the substituent is larger in size than the naturally occurring 3' hydroxyl group, compared to the control polymerase.

7. A nucleic acid molecule encoding a polymerase according to any the preceding claims.

8. An expression vector comprising the nucleic acid molecule of claim 7.

9. A method for incorporating nucleotides which have been modified at the 3' sugar hydroxyl such that the substituent is larger in size than the naturally occurring 3' hydroxyl group into DNA comprising the following substances (i) a polymerase according to any one of claims 1 to 6, (ii) template DNA, (iii) one or more nucleotides, which have been modified at the 3' sugar hydroxyl such that the substituent is larger in size than the naturally occurring 3' hydroxyl group.

10. Use of a polymerase according to any of the claims 1 to 6 for DNA sequencing, DNA labeling, primer extension, amplification or the like.

11. Kit comprising a polymerase according to any of the claims 1 to 6.
